# EUROPEAN PATENT APPLICATION

(11) **EP 4 226 890 A1**
(43) Date of publication of application: **16.08.2023**
(21) Application number: 22156802.5
(22) Date of filing: 15.02.2022
(51) Int. Cl.: A61C 7/08, A61C 19/06

(54) **DENTAL APPLIANCE FOR INTRA-ORAL DELIVERY OF ONE OR MORE AGENTS**

(71) Applicant: Bottmedical AG, 4057 Basel (CH)
(72) Inventor: Osmani, Bekim, 4056 Basel (CH); Töpper, Tino, 79115 Freiburg (DE); Dard, Elise, 4055 Basel (CH)
(74) Representative: Mertzlufft-Paufler, Cornelius

(57) **Abstract**

For improving control of the release of an agent (7) from a dental appliance (1), it is proposed to accurately control the formation and geometry of a multitude of micro-reservoirs (3), which are formed in a core (4) of the appliance (1) during the fabrication of the same, preferably within a limited region of interest (13). This approach allows accurate control of the rate at which the micro-reservoirs (3) release the agent (7) into the mouth and the total dosage that will be delivered to the patient by the appliance (1). The approach may be carried out using a computer-implemented method that enables rapid fabrication of a series (30) of such appliances (1) which are each personalized to the specific needs of a patient

## Description

The present disclosure concerns a dental appliance to be worn on teeth for intra-oral delivery of one or more agents to a patient. The appliance comprises a solid core, which may be preferably made of a thermoplastic material that can be thermoformed. In particular, the appliance may be fabricated by Tamil forming the core into a patient-specific shape.

The disclosure further concerns a series of such appliances, a process for fabricating a dental appliance and a method for reloading (i.e., re-equipping) such an appliance with a releasable agent, for example a flavor, a drug, or an antimicrobial agent.

Finally, the disclosure concerns a computer implemented method for producing a serious of different dental appliances.

It is well-known that releasable agents such as antimicrobial agents can be loaded by diffusion processes into porous superficial layers of a dental appliance. When the user is wearing such an appliance in his mouth, the agent may be released from the appliance to provide protection against inflammation of the teeth and gums. Loading such agents into a dental appliance can also be beneficial for keeping the appliance hygienically clean, i.e., free from dangerous pathogens.

Starting out from this background, the invention aims at providing dental appliances and associated methods that can provide substantial benefits for the end-user. In particular, it is an option act of the invention to provide means that allow personalized delivery of an agent to a patient's mouth that is tailored to the specific needs of that patient.

In accordance with the present invention, a dental appliance is provided according to claim 1, which achieves the aforementioned objectives. In particular, the invention proposes a dental appliance as introduced at the beginning, which, in addition, is characterized in that, within a region of interest, the core features a multitude of micro-reservoirs, which are (or more precisely have been) formed in the core.

Preferably, the micro-reservoirs may be formed in an outer surface of the core.

Each of the micro-reservoirs may have sub-mm dimensions, in particular a diameter below 1 mm or preferably below 0.2 mm. This approach is highly beneficial for accurate control of the release rate within the defined region of interest (ROI) of the dental appliance, because the micro-reservoirs can be loaded with a releasable agent. This may be achieved by letting the agent diffuse from a source (e.g., a reload liquid containing the agent) into the micro-reservoirs.

In addition, the proposed approach - for example through variation of the spatial density (number of micro-reservoirs/ cm²) and/or depth and/or diameter of each micro-reservoir - allows application/delivery of a localized (i.e., only within the ROI) and patient-specific dosage of at least one releasable agent to a ROI within the mouth of a patient. When the patient is wearing the appliance, the ROI is defined by the location of the micro-reservoirs on the appliance, for example at a particular tooth that has undergone surgery and is thus susceptible to a possible inflammation.

As mentioned, the proposed micro-reservoirs can be loaded with a defined amount of releasable agent prior to use of the dental appliance in the mouth of a patient. Moreover, the rate at which the micro-reservoirs release the agent in the mouth to a patient, which will define the dosage delivered to the patient, can be accurately controlled by controlling the geometry of the micro-reservoirs during the fabrication of the dental appliance. A precise dosage of the agent can thus be delivered to the ROI over prolonged time of several hours (during which the patient is wearing the dental appliance on his teeth).

The dosage to be delivered to an individual patient can be readily and comfortably controlled by controlling the geometric parameters of the micro-reservoirs formed in the core, in particular the diameter and/or depth of each micro-reservoir (and thereby its volume).

In addition, the choice of a possible filling material (filling up the micro-reservoirs) and the use of a diffusion barrier can be used to fine-tune the release rate of the agent, when the dental appliance is worn in the mouth, as will be detailed below.

Using a high number of micro-reservoirs also has great advantages, in particular when the dental appliance is thermoformed after the micro-reservoirs have been formed in the core, because in this particular case, the risk of affecting the mechanical stability of the core (which would be the case when using only two or three larger reservoirs) will be minimized. The definition of the reservoirs with microscopic dimensions thus helps to maintain the mechanical stability of the core during thermoforming of the dental appliance.

The appliance itself may be designed (in particular as a dental splint) such that the core forms a plurality of cavities (at least two) shaped to receive teeth of a mouth of a patient (patient-specific design). The micro-reservoirs may be generally designed such that they can receive (in particular through a diffusion barrier) and store one or more agents and later release the one or more agents to the patient, more precisely to the ROI of the mouth that is adjacent to said ROI of the appliance, in which the micro-reservoirs are located.

More details of possible embodiments of such a dental appliance are described in the sub-claims and will now be presented:
For example, each micro-reservoir may be formed by an air pocket of sub-mm size, for example with a diameter of at least 10 µm, preferably of at least 50 µm, in particular of at least 100 µm.

Importantly, each air pocket may have been actively formed at a pre-defined location using a separate fabrication step. In other words, an air pocket in the meaning of the invention is not simply a pore within a porous material but a geometrically well-defined and intentionally created reservoir at a particular location. This reservoir/pocket may take up an agent, store it and later release it to the patient.

The active control of the geometry of the air pocket by applying a separate fabrication step (e.g. laser processing, 3D-printing, hot embossing, micro-machining, molding or other additive or subtractive processes) is crucial for obtaining precise control of the geometry and thereby control of the amount of agent and resulting release rate that can be obtained with each micro-reservoir. This control is particularly important for enabling personalization of the micro-reservoirs for the specific needs of a patient, as will be explained in greater detail below.

Alternatively, each micro-reservoir may be filled, at least partly, with a filling material that is capable of absorbing and releasing an agent, such as a flavor molecule, an antimicrobial or a drug. In the final appliance (directly before use), the filling material may be loaded with such an agent.

The filling material may preferably fill up the respective micro-reservoir completely. Using this approach, the filling material can be firmly linked to the micro-reservoir, in particular when the micro-reservoir increases in diameter with increasing depth (e.g., in the shape of a pear).

According to another embodiment, the micro-reservoirs may be covered by a cap layer. Preferably the cap layer can comprise or preferably be made up entirely of cellulose acetate butyrate (CAB). The cap layer may cover an area of the core that stretches over a distance that is at least ten times an average distance between two of the micro-reservoirs which are adjacent to each other. Preferably, the cap layer can be a layer of uniform thickness.

The cap layer may have a thickness of less than 50 µm, preferably of less than 25 µm. Such a design of the appliance allows to maintain a high wearing comfort for the user, because the overall dental appliance can remain thin and flexible.

According to one particular embodiment, the cap layer can be made up by the filling material. In this case, the cap layer may form an outer surface of the dental appliance. In addition, the cap layer may be such that the agent can be loaded into a surface area that is defined/covered by the outer cap layer. In this embodiment, the filling material may thus form a uniform outer cap layer that connects the micro-reservoirs with each other and fills them.

The solid core of the appliance can be made from Polyethylenterephthalat (PET), preferably from glycol modified Polyethylenterephthalat (PETG). In this case, a suitable filling material is a cellulose-based filling material. Preferably the filling material may comprise cellulose acetate butyrate (CAB). This material system allows accurate control of the release rate of a number of particularly small-sized agents.

As already mentioned, the micro-reservoirs may be formed as micro-cavities and may be micro-sized, i.e. with diameters below 1 mm. For example, the micro-reservoirs can have the shape of a dead hole with a bottom of each micro-reservoir being formed by the material of the core.

The multitude of micro-reservoirs may comprise, for example, a number of at least 20, at least 50 or even at least 100 micro-reservoirs. A typical spatial density of the micro-reservoirs may be in the range [1 - 100] micro-reservoirs / cm². Moreover, the micro-reservoirs may be arranged within the ROI in a regular or irregular pattern.

The depth t of each micro-reservoir may also be of sub-mm size, preferably in the range of t = [50 - 800] µm.

As mentioned, the multitude of micro-reservoirs may be located within a particular region of interest (ROI) of the dental appliance, i.e., they do not need to be spread over the full outer surface of the core. This approach allows concentrated and controlled release of the agent within the ROI, e.g. at a tooth location, where the user wearing the dental appliance has obtained an implant that is susceptible for inflammation. In such a case, the dental appliance can provide a tailor-made protection against inflammation by releasing a drug or an antimicrobial into the ROI, where it is needed.

The micro-reservoirs can each have a diameter of less than 0.5 mm, preferably of less than 0.2 mm. Most preferably their depth can be at least 5%, preferably at least 25%, of a thickness of the core.

Furthermore, it is beneficial for achieving a prolonged time of delivery of the agent if the depth of each micro-reservoir is larger than 0.05 mm, preferably larger than 0.2 mm or even larger than 0.5 mm. In other words, the micro-reservoirs may each offer an aspect ratio AR = depth/diameter of AR ≥ 0.2, preferably of AR ≥ 0.5, such that the depth of each micro-reservoir amounts to at least 20%, preferably at least 50%, of the diameter of the respective micro-reservoir.

According to one embodiment, the micro-reservoirs may have been patterned into the core after forming the core (but possibly before thermoforming the core into its final shape). This may be done preferably using an ablative process, most preferably an ablative laser-process, which removes the material of the core at the location of each micro-reservoir.

Alternatively, the micro-reservoirs may be implemented also with non-ablative processes, for example by hot embossing the micro-reservoirs into the core, for example after forming the core as a foil.

In all of these variants, the mentioned filling material (if employed) can be filled into the micro-reservoirs after formation of the micro-reservoirs, for example by using a blade process or roll-to-roll-process or a lamination technique. Finally, the desired final dental appliance shape may then be obtained by thermoforming the core (in particular in the shape of a core foil) with filled micro-cavities using conventional techniques.

The invention may also be implemented by forming the micro-reservoirs together with the core in a single fabrication step. This may be done, in particular, using: (i) an additive manufacturing technique such as 3D-printing, in particular such that the micro-reservoirs are embedded in the core; or (ii) a forming technique such as hot embossing, in particular wherein the core features multiple layers of different materials; or (iii) the micro-reservoirs may have been formed in a separate outer, in particular 3D-printed, layer of the core that is formed on a solid inner layer of the core.

According to another embodiment, the cap layer, which may be an outer layer of the appliance, is different from the filling material. In this case, the cap layer may cover (and thus close) each of the micro-reservoirs, in particular in such a way that the agent can be loaded and released from the filling material located in the micro-reservoirs only through the cap layer. This approach can thus provide accurate control of the release rate of the agent through the choice of material and choice of thickness of the cap layer.

In more detail, the cap layer may offer a diffusion rate with respect to an agent (to be delivered to the patient and) that is embedded into the filling material that is at least 50% lower than the corresponding diffusion rate of the filling material. In this case, the cap layer may effectively form a diffusion barrier that limits the release rate of each of the micro-reservoirs.

For example, if the diffusion rate of the cap layer is 50% lower than that of the filling material, this can result in a release rate of the micro-reservoir that is reduced by roughly 50% as compared to the case of using no diffusion barrier / no cap layer. As a result, the useable period of time in which the appliance delivers a minimum dosage of the agent may be prolonged from 4 to 6 hours, as an example, thus providing prolonged protection for the patient.

In this variant, the release rate of each micro-reservoir can thus be accurately controlled by choice and dimensioning (in particular regarding the thickness) of the cap layer.

The embedding of the agent into the filling material located in the micro-cavities can be achieved by soaking the dental appliance in a reload-liquid containing the agent at a defined concentration. The agent may thus diffuse into the filling material over time. Afterwards, when the dental appliance is worn on the teeth of a user, the agent may be released from the micro-reservoirs over a prolonged time at a relatively moderate release rate, since the cap layer inhibits the diffusion of the agent out of each of the micro-reservoirs.

According to a preferred embodiment, the cap layer may be formed as a diffusion barrier from a cellulose-based thermoplastic material, preferably comprising cellulose acetate butyrate (CAB). This material should offer short length of its molecular chains to achieve the desired low diffusion rate.

According to another embodiment, the cap layer may be different from the filling material, and it may cover each of the micro-reservoirs. Furthermore, for each micro-reservoir, a corresponding release hole, preferably with a diameter of less than 20 µm, may be formed in the cap layer. The agent can then be release from the reservoir through the release hole, which offers a well-defined cross-sectional area for this transfer. In such an embodiment, the cap layer can be even impermeable to the agent that is embedded into the filling material. Through the release holes, the agent can be released from the filling material located in the respective micro-reservoir at a defined and lowered release rate, as compared to a direct release from the reservoir.

The agent may have been embedded into the filling material prior to covering the micro-reservoirs with the outer cap layer. However, a preferred approach is to load the agent into the filling material after completing fabrication of the dental appliance. In the latter case, the agent can be transferred through the cap layer and/or through the release hole (if employed) into the respective micro-reservoir.

One particular advantageous embodiment suggests that a cross-sectional area of the release hole is smaller than a micro-release-opening that is defined by the micro-reservoir (its outermost opening to the outside world). To achieve a low release rate and thus the ability to release the agent from the micro-reservoir over a prolonged period of time, it is of advantage if the cross-sectional area of the release hole is less than 5 times, preferably less than 10 times, the cross-sectional area of the micro-release-opening.

In detail, each micro-reservoir may thus offer a micro-release-opening of defined dimensions located in the outer surface of the core. Preferably the micro-release-openings may be of same cross-sectional area. In this case, each micro-reservoir can produce the same release-rate of the agent in an oral cavity when fully loaded (saturated) with the agent.

The release rate may be measured for example as the amount of agent (in mg) that is released from one of the micro-reservoirs within a defined period of time (in minutes/hours), in a defined ambient (in particular at a defined humidity).

According to a preferred embodiment, all of the micro-reservoirs may show a constant depth and/or volume. In this case, each micro-reservoir can be loaded with a same maximum amount of a releasable agent and/or each micro-reservoir can thus offer the same amount of maximum release dosage of the agent.

The release dosage may be measured for example as the amount of agent (in mg) that can be loaded into and released from one of the micro-reservoirs within a limited period of time, e.g., 12 hours. Preferably, all micro-reservoirs may show identical dimensions, and may be filled with the same filling material. In this case, they will offer the same amount of releasable agent when fully loaded with a commonly used agent.

According to another embodiment, the filling material may be a thermoplastic material that can be thermoformed together with the core to form the final dental appliance.

In addition, or alternatively, the filling material may also be a porous material featuring microscopic, sub-micrometer-sized, voids.

To ease fabrication of the appliance by thermoforming, one particular embodiment suggests that a melting temperature of the filling material is lower than a glass transition temperature of the material used for the core. Moreover, said melting temperature may be equal or lower than the melting temperature of the cap layer (if employed). Such a material choice will result in enough mobility of the molecular chains of both core and filling material such that efficient thermal interlocking can be achieved through thermal fusion (sometimes referred to as "thermal welding"). This has the advantage, that the core can be thermally fused with the filling material during thermoforming of the dental appliance, which results in excellent anchoring of the filling material within the core.

For meeting the objectives mentioned at the beginning, the invention further proposes a series of dental appliances (each intended for intra-oral delivery of one or more agents to a patient). Each of the appliances of that series may have features as described before, in particular according to claim 1, i.e., each appliance may have a multitude of micro-reservoirs formed in the core of the respective dental appliance. The series is characterized in that the dental appliances of the series are all made from common materials and share a common solid core. Preferably, the dental appliances may employ the same type of micro-reservoirs fabricated in the same way. In addition, the dental appliances differ from each other in at least one geometrical design parameter of the individual micro-reservoirs. Such a design parameter, which may characterize each one of the micro-reservoirs and/or the arrangement of the micro-reservoirs in the core, may be a spatial density and/or an (e.g., average) depth and/or a respective volume of the micro-reservoirs.

By arranging the micro-reservoirs in a certain spatial density (measure as number of micro-reservoirs/cm²), it is possible to define a maximum areal release rate in (mg/cm²)/hour, that can be delivered in/to the ROI.

In addition, said geometrical design parameter of the individual micro-reservoir employed in the respective appliance of the series may define the respective total amount of releasable agent (measured in mg over a period of 6 hours for example) either per micro-reservoir or for all micro-reservoirs of the appliance and/or the maximum initial release rate of an agent (in mg/hour) that can be delivered by the appliance as soon as it is fully loaded / saturated with an agent.

It is also possible to alter other design parameters such as the diameter of the respective release hole employed with a micro-reservoir (in case a cap layer is used) and/or the thickness and/or material of the cap layer (if employed).

All of these approaches allow tailoring of the amount of agent and its release rate to the needs of a specific patient. The series can thus comprise a number of different appliances, each designed/tailored for a patient-specific application case. This approach can be supported by a computer-implemented-method, that will now be described in more detail.

For enabling rapid delivery of a dental appliance that is personalized to the specific (in particular medical) needs of a patient, the invention proposes a computer-implemented method for producing a series of different dental appliances, which may have features as just explained. This method comprises the following steps:
Step (1): specifying a number of m different numerical values for at least one agent release parameter for a particular releasable agent (to be delivered by the appliance to a patient) in a digital data set. Such an "agent release parameter" may be, for example, (a) a maximum initial release rate in mg/hour and/or (b) a total maximum release amount in mg over a time of six hours and/or (c) a maximum areal release rate in (mg/cm²)/hour.
Step (2): calculating a set of m different design parameters for defining a set of m different micro-reservoir-arrangements. This may be done using a numerical and/or empirical model. Such a model can be derived from pre-experiments in which the release rate and/or total amount of releasable agent per micro-reservoir have been measured for a particular micro-reservoir design. The simplest way of fine-tuning the release rate of a particular appliance can be to alter the spatial density and/or number of micro-reservoirs per appliance within the series of appliances. Further possible design parameters that define the micro-reservoir arrangement may be: diameter and/or depth of each micro-reservoir; volume of each reservoir; spatial density of the reservoirs; diameter of release hole (if employed); choice of material and thickness of cap layer (if employed); etc.
Step (3): producing said series of m different dental appliances, wherein each of the m dental appliances features a micro-reservoir-arrangement according to one of the m different design parameters. In other words, each of the m micro-reservoir arrangements may be different and tailored for the need of a patient at a certain point in time.

As a result, each of the m different dental appliances of that series can meet the respective specified release parameter (as documented in the digital data set).

In this method, a computer may be employed to process large data sets and to automatically calculate and deliver the necessary design parameters for defining the micro-reservoirs of each appliance of the series. The design parameters can also be used as digital input to be delivered to a machine (e.g. a laser device) that forms the micro-reservoirs in the respective cores.

For each appliance of the series, the generation of the respective specific micro-reservoir arrangement (in 2D or 3D) can comprise locating the respective micro-reservoirs in a specific ROI that has been specified beforehand. The computer-implemented method can thus deliver a digital data set specifying the micro-reservoir-arrangement, e.g., the pattern of arrangement (and hence spatial density) of the micro-reservoirs and/or specific geometrical design parameters, such as a spatial density and/or an average depth and/or a respective volume, of the individual micro-reservoirs of the arrangement for each appliance of the series.

These data sets can then be used for fabricating the different appliances, in particular using an ablative process such as laser ablation for defining the micro-reservoirs in the core, in particular in a core foil from which the core is thermoformed.

Following this approach, one embodiment of this method proposes that the digital data set (which specifies the m different numerical values for the at least one agent release parameter) comprises (i) a set of patient-specific values, each being based on the specific medical need of a single patient, in particular as specified by a treatment instruction for that patient and/or (ii) a subset of N treatment-specific-values, each based on a specified dosage of the at least one agent to be delivered to a particular patient within a specified time interval.

Such values, for example a total daily dosage to be delivered to a particular patient over a duration of several weeks, may be laid out in a treatment plan for that patient. By wearing (and reloading) daily a first dental appliance according to the invention and produced with the computer-implemented method over a first period of time, the patient can safeguard that he receives a desired first dosage. After some weeks, the patient may switch to a second dental appliance of the series which offers a lower second dosage of the agent, and he may wear and reload this second appliance repeatedly over a second period of time. Following this concept, the dosage delivered to the patient can be accurately controlled stepwise with minimal effort for the patient, as he only needs to wear the right appliance designed and produced for the dosage to be delivered within a specific time interval. In this first scenario, a single patient can thus alter the dosage delivered to his mouth by changing the appliances (taken from said series) which he wears on his teeth, and thus conveniently follow a treatment plan that has been recommended by a doctor, for example.

In a second scenario, the dosage to be delivered to a number of m patients can be specified and the method can help in rapidly delivering custom-made and personalized dental appliances for each of those patients that meet the requirements concerning release rate and dosage with respect to a particular agent to be delivered to a specific patient.

The computer-implemented method proposed here thus uses input such a patient-specific treatment instructions or treatment plans to derive a suitable data set that can then be used to fabricate a series of dental appliances, which can deliver the agent of interest at the desired dosage to a specific patient during the time interval, in which the patient is wearing the appliance in his mouth (e.g. for several hours over night).

Finally, a process for fabricating dental appliances as described before is proposed. This process is characterized in that a core of the dental appliance is first formed as a core foil, a multitude of micro-reservoirs is/are defined in an outer surface of the core (foil) using (i) an ablative process, preferably an ablative laser-process, which removes the material of the core at the location of each micro-reservoir; or (ii) a forming process, such as hot embossing; or (iii) an additive process, for example molding or 3D-printing of an additional outer layer of the core. Of course, it is highly advantageous if the micro-reservoirs defined by that process have features as described before or as specified in the claims directed towards a dental appliance.

The final dental appliance may then be obtained by thermoforming the core (foil) with the already pre-formed micro-reservoirs. Additionally, the final dental appliance may be trimmed in size afterwards, as is known in the art.

Furthermore, as has been explained in detail before, the micro-reservoirs may be filled with a common filling material that is capable of absorbing and releasing an agent such as a flavor molecule or a drug. This may be done prior or after thermoforming of the appliance.

In addition, a cap layer may be deposited onto the core that covers the micro-reservoirs. This may also be done either prior or after the thermoforming of the appliance. As mentioned before, the cap layer may close the micro-reservoirs and/or it may form a diffusion barrier (i.e. slowing diffusion down) or it may even by a layer that is impermeable for the agent (thus preventing any diffusion through the cap layer).

Finally, for each micro-reservoir, a corresponding release hole may be formed in the cap layer. This may, preferably, be done using an ablative laser process. This approach is particularly suited when the cap layer is impermeable for the agent.

The laser cutting of the release holes may be done by considering dedicated alignment marks / fiducials; the latter may be patterned together with the micro-reservoirs into or onto the core foil, more precisely into a master foil from which pieces may be cut out to serve as the core foil. Using this approach, each release hole can be accurately positioned over the respective micro-reservoir.

The micro-reservoirs may be patterned into or onto the core with a constant depth and/or volume, preferably such that each micro-reservoir produces the same release-rate and/or release-dosage of the agent in an oral cavity when fully loaded with the agent. Preferably, the multitude of micro-reservoirs may be only defined in at least one spatially limited region of interest (ROI). The at least one ROI may cover less than 10% or even less of an outer surface of the final dental appliance, for example.

Finally, the invention also aims at improving the ease of use of the dental appliances described so far. For this purpose, a method is proposed for reloading a dental appliance (which may have features as described before) with a releasable agent such as a flavor, a drug, or an antimicrobial agent.

The method is characterized in that the dental appliance is immersed in a reload-liquid containing the agent. Different from the state-of-the-art, however, the reload-liquid is heated to an elevated temperature of at least 55°C, while the dental appliance is immersed in the reload-liquid.

Furthermore, it is suggested to employ an electrically heatable reload-station which provides an immersion basin for taking up and heating up the reload-liquid. The electrical heating itself may be implemented by resistive heating or by applying electrically induced ultrasound (ultrasonic waves) to the reload-liquid, to name two possible implementations.

Due to reasons of health protection and effective regulations, the agent can only be contained in the reload-station at a maximum concentration that is not harmful to the patient. This maximum concentration thus normally limits the speed at which the reloading takes place. In other words, the time required to fully reload the micro-reservoirs of an appliance will be limited by that maximum concentration. The proposed approach has the great advantage of significantly reducing this time, because the diffusion of the agent into the reservoirs, will be accelerated as compared to diffusion at room temperature, in particular when employing a cap layer in the dental appliance.

Examples of the present invention will now be described in more detail with reference to the accompanying drawings, where features with corresponding technical function are referenced with same numerals even when these features differ in shape or design:
- Fig. 1: is an illustration of a fabrication of a dental appliance by thermoforming according to the state-of-the-art,
- Fig. 2: shows an illustration of fabricating a dental appliance according to the invention from a thermoplastic core foil,
- Fig. 3 to 10: illustrate simplified and schematic cross-sections through dental appliances according to the invention,
- Fig. 11: illustrates the effect of thermoforming on a pre-defined micro-reservoir in the form of a microscopic air pocket formed in the core of a dental appliance according to the invention,
- Fig. 12: illustrates the effect of thermoforming on a pre-defined micro-reservoir in the form of a microscopic air pocket formed in the core of another dental appliance according to the invention,
- Fig. 13: illustrates another possible design of a dental appliance according to the invention,
- Fig. 14: illustrates a series of dental appliances according to the invention, which have been fabricated using a computer-implemented method according to the invention, and
- Fig. 15: illustrates the use of a reload-station according to the invention designed for reloading or initially loading an agent into any suitable dental appliance.

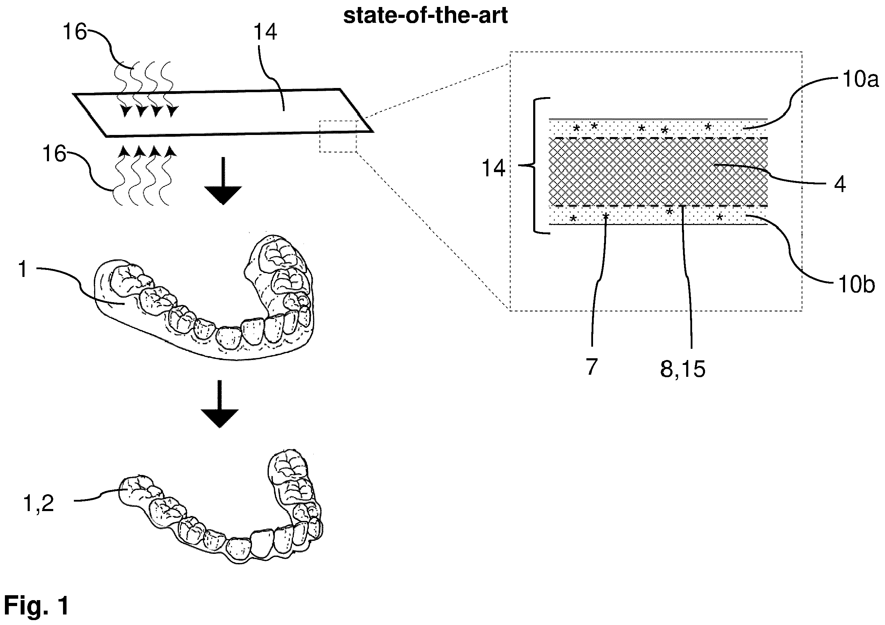

Figure 1 displays a process as known in the state-of-the-art for fabricating a dental appliance 1 from a thermoplastic foil 14. The foil 14 comprises a core 4 and two outer cap layers 10a and 10b. The layers 10a, 10b are softer than the core 4 and used to provide an improved wearing comfort for the user. Under the impact of heat and pressure 16, the appliance 1 can be thermoformed from the core foil 14 and trimmed in size afterwards.

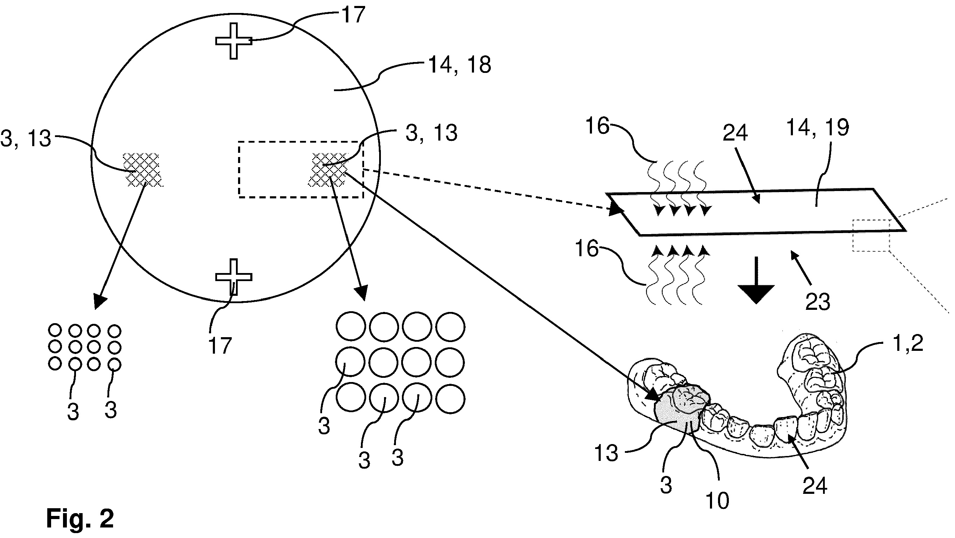

Figure 2 shows an illustration of the comparable fabrication of a first dental appliance 1 according to the invention:
Again, a thermoplastic foil 14, namely a master foil 18, is employed but prior to thermoforming, a multitude of sub-mm sized micro-reservoirs 3 are formed in the foil 14 within a particular region of interest 13 (ROI) using an ablative lasering process. The micro-reservoirs 3 are arranged as a 2D-array and the location of each individual micro-reservoir 3 is controlled by considering fiducials 17 on the master foil 18. Afterwards the master foil 18 is cut into single pieces 19, and from each of the pieces 19 a dental appliance 1 is obtained by thermoforming the piece 19. The location of the micro-reservoirs 3 within the piece 19 is thus also well-known.

As the micro-reservoirs 3 have been patterned in the outer surface 24 of the foil 14, the micro-reservoirs 3 of the final appliance 1 are facing the gums and inner oral cavity of the patient, when the patient is wearing the dental appliance 1 on his teeth. As illustrated in figure 2, arranging the micro-reservoirs 3 within a certain area of the master foil 18 has resulted in the micro-reservoirs being located in a region of interest (ROI) 13 that is located at the one-before-last molar of the patient.

The micro-reservoirs 3 in Figure 2 can be such that they are formed with the same diameters and same depth and show a diameter of less than 0.2 mm and a depth of 0.1 mm, resulting in an aspect ratio of AR = depth/diameter > 0.5.

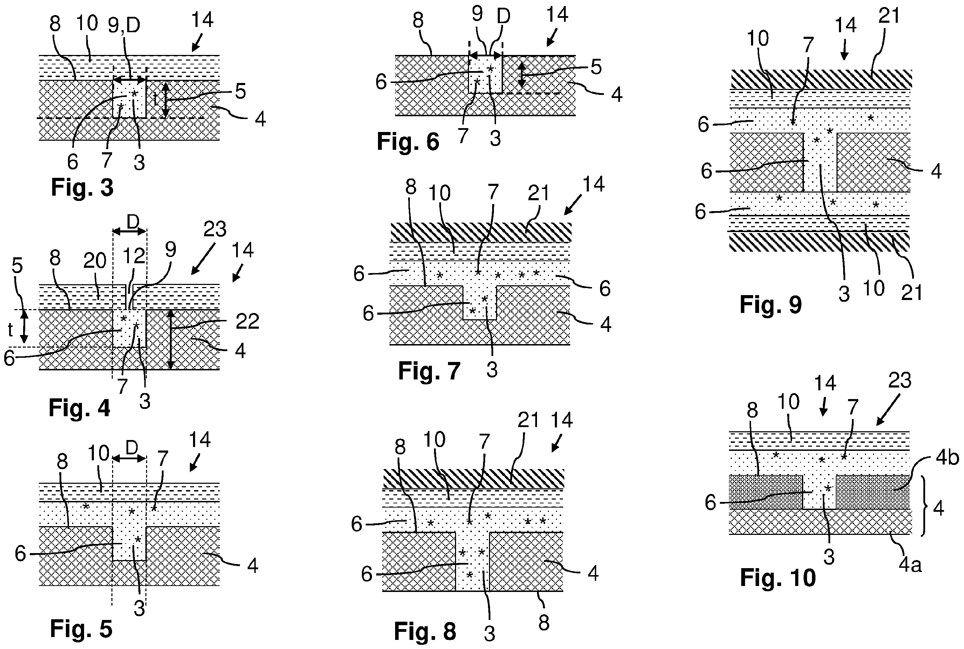

Figures 3 to 10 provide further examples of possible embodiments of dental appliances 1 according to the invention. In all examples according to the invention, a material is used for the core 4 which shows a negligible diffusion rate for the agent 7, such that the core 4 can be considered impermeable for the agent 7. Shown are simplified and schematic cross-sections through the foil 14, from which the final appliance 1 will be thermoformed. Each appliance 1 will thus feature a solid core 4, in which a multitude of micro-reservoirs 3 have been patterned, although only one of these micro-reservoirs 3 is illustrated. In the shown examples, each micro-reservoir 3 is entirely filled up with a filling material 6, which is capable of storing and releasing an antimicrobial agent 7.

In the example of figure 3, the micro-reservoir 3 is formed as at dead hole, with the core 4 forming a bottom of that hole. The micro-reservoir 3 is covered and closed by a cap layer 10 which forms a diffusion barrier for the agent 7 contained in the filling material 6, because the diffusion rate of the agent 7 is much lower in the cap layer 10 as compared to the filling material 6.

Compared to the example of Figure 6, in which no such cap layer 10 is employed (such that the micro-reservoir 3 forms a micro-release-opening 9 of diameter D in the outer surface 8 of the foil 14 and hence the final core 4 of the appliance 1), the release rate of the design shown in Figure 3 will be much lower. As a result, the amount of time during which a significant amount of the agent 7 can be released from the appliance 1 will be prolonged in the example of Figure 3 as compared to Figure 6, at least when using the same materials and agent 7.

Figure 4 illustrates another possible approach, in which a dedicated release hole 12 is employed, which has been formed in the cap layer 10. In this example, the cap layer is a layer 20 that is impermeable to the agent 7. Accordingly, the agent 7 can only be loaded into the micro-reservoir 3 and released from the same through the release hole 12. The diameter of the release hole 12 thus provides convenient control of the release rate, because it defines the cross-sectional area through which the transfer of the agent 7 can happen.

The difference between the design of figure 5 compared to that of figure 3 is that the filling material 6 not only fills up the micro-reservoir 3 but also covers at least part of the outer surface of the core 4. As illustrated by the star symbols, the agent 7 can thus not only be embedded into the micro-reservoirs 3 but also in the superficial part of the filling material 6 layer just below the outer cap layer 10. It is obvious that the agent 7 can freely diffuse within the filling material 6.

In the example of Figure 7, an additional outermost layer 21 has been added as compared to the design of Figure 5. This layer 21 can swell up in water and is used for the sole purpose of improving the wearing comfort of the dental appliance 1. In this design, the cap layer 10 is therefore not an outermost layer but an inner layer off the muti-layer structure displayed.

The example of figure 8 differs from that of figure 7 in that the micro-reservoir 3 is no longer formed as a dead hole but rather as a through hole. This has the advantage that the micro-reservoir 3 can be loaded with the agent 7 from the upper and lower side.

In the more sophisticated design of figure 9, an additional cap layer 10 and additional outermost layer 21 have been added to the lower side of the structure, which may form the inner surface of the final dental appliance 1 that is resting on the teeth.

Figure 10 illustrates another possible design of a dental appliance 1 according to the invention in which the core 4 is designed as a multi-layer structure with a solid inner core 4a on which an outer core layer 4b has been 3D-printed. In this design, the micro-reservoirs 3 have thus been formed in a separate outer layer 4b of the core 4 while fabricating the core 4 using an additive manufacturing process, namely 3D-printing. Afterwards, the micro-reservoirs 3 have been filled up with a filling material 6, which also covers the micro-reservoir 3. Finally, a cap layer 10 was added to form a diffusion barrier.

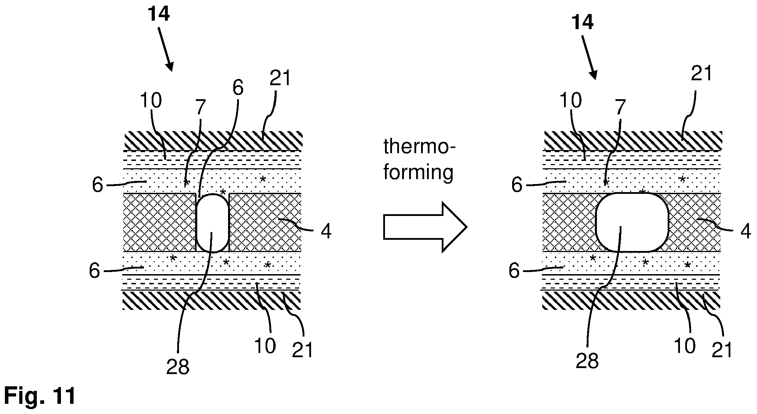

Figure 11 illustrates the example of a micro-reservoir 3 according to the invention formed as an air pocket 28 with a diameter of 0.2 mm in a solid core 4 of a thermoplastic material. This was achieved simply by drilling a hole through the core 4 and applying a filling material 6 using blade coating processes on both sides of the core 4. As can be seen in figure 11 on the left, this process does not fill up the micro-reservoir 3 completely, such that said air pocket 28 remains in the core 4. After adding cap layers 10 and soft outermost layers 21 on both sides of the core foil 14, the structure is ready for thermoforming. On the right of figure 11, the result of the thermoforming is displayed. As visible, the air pocket 28 has expanded due to the elevated temperatures during thermoforming and sort of fused with the material of the core 4, which was highly softened during the thermoforming. As a result of this change in geometry, the cross-section through which diffusion can happen has been reduced leading to a lower release rate.

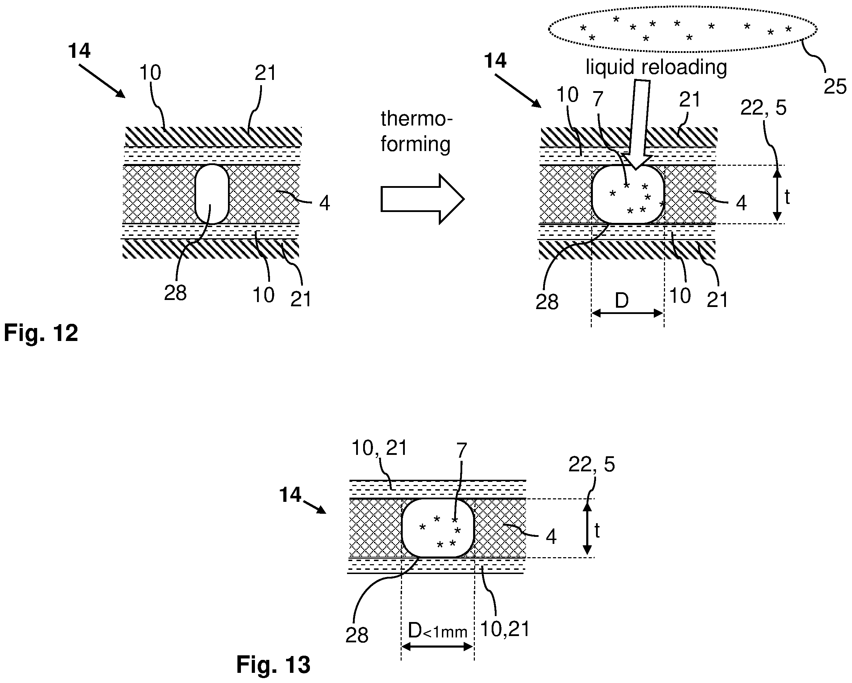

Figure 12 illustrates another design, in which an air pocket 28 is used as a micro-reservoir 3 that is embedded in the core 4 of the dental appliance 1. After thermoforming of the core foil 14, the micro-reservoir 3 has been stretched and enlarged. By immersing the dental appliance 1 into a reload-liquid 25 which contains the agent 7 in a high concentration, the agent 7 can be loaded into the micro-reservoir 3 by letting the agent 7 diffuse through the outermost layer 21 and the cap layer 10. In this case, the agent 7 and other ingredients of the reload-liquid 25 can fully replace the air in the pocket 28 until the pocket 28 / the micro-reservoir 3 is fully loaded. This process may be repeated (= reloading of the appliance 1) after the dental appliance 1 has been used by a user and has released most of the amount of agent 7 stored in the multitude of micro-reservoirs 3.

As figure 13 displays, air pockets 28 may also be used in a simple design in which the core 4 / the micro-reservoir 3 is only covered by a single outermost cap layer 10 (for example on both sides as displayed).

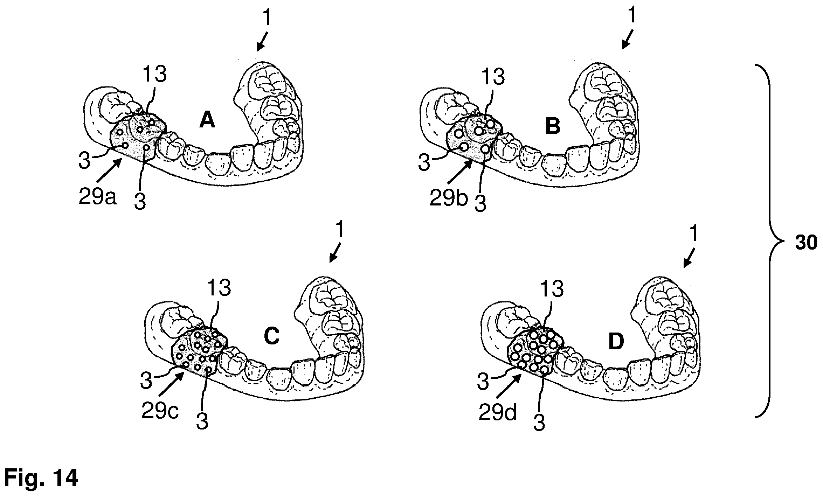

Figure 14 illustrates a series of four different dental appliances 1 A-D, which are all fabricated from the same materials using same processes and are all tailored in shape to the teeth of one patient. The appliances 1 differ, however, in the arrangement 29 of the micro-reservoirs 3 with respect to spatial density and/or individual size of the micro-reservoirs 3. As indicated (for example compare A to B), for changing the release rate and/or the maximum dosage of the agent 7 delivered by one of the appliances 1, it may be sufficient to alter a geometrical design parameter of the micro-reservoirs 3 such as their diameter (and thereby the size, volume and hence the amount of agent in mg that can be stored in each micro-reservoir 3) while maintaining a particular pattern of the 2D-arrangement of the micro-reservoirs 3.

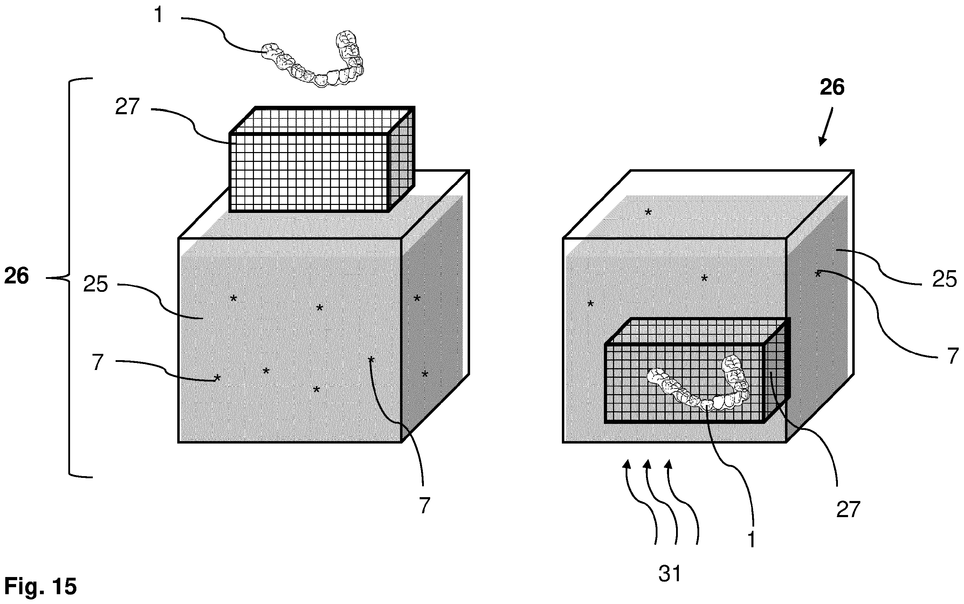

Figure 15 finally illustrates the use of a reload-station 26, according to the claim describing the process proposed by the invention for rapidly reloading any suitable dental appliance 1 capable of storing a releasable agent 7 with that agent. As illustrated, the reload-station 26 offers an immersion basin 27 for taking up and heating up electrically a reload-liquid 25 containing the agent 7. Due to the elevated temperatures, the diffusion process is accelerated resulting in a greatly reduced reload-time.

In summary, it is proposed to accurately control the formation and geometry of a multitude of micro-reservoirs 3, which are actively formed in a core 4 of a dental appliance 1 during the fabrication of the same, preferably within a limited region of interest (ROI) 13. This approach allows accurate control of the rate at which the micro-reservoirs 3 release the agent 7 into the mouth of a patient and the total dosage that will be delivered to the patient by the appliance 1. The approach may be carried out using a computer-implemented method that enables rapid fabrication of a series 30 of such appliances 1, which are each personalized to the specific needs of a single patient or a group of patients.

### List of reference numerals

- 1: dental appliance
- 2: dental splint
- 3: micro-reservoir
- 4: core
- 5: depth (of 3)
- 6: filling material
- 7: agent
- 8: outer surface (of 4)
- 9: micro-release-opening (of 3 in 8)
- 10: cap layer (covering 4 and 3)
- 11: surface area (covered by 10)
- 12: release hole (formed in 10)
- 13: region of interest (on 4, in which 3 are located)
- 14: (thermoplastic) foil
- 15: interface (e.g. between 4 and 10)
- 16: heat, pressure
- 17: fiducial
- 18: master foil
- 19: piece (cut out of 18)
- 20: impermeable cap layer
- 21: outermost layer (e.g. for improving the wearing comfort of 1) - may swell up in water
- 22: thickness (of 4)
- 23: inner surface (of 1)
- 24: outer surface (of 1)
- 25: reload-liquid (containing 7)
- 26: reload-station
- 27: immersion basin
- 28: air pocket
- 29: micro-reservoir-arrangement
- 30: series of dental appliances
- 31: electrical heating (resistive heating or ultrasound)

## Claims

1. **Dental appliance** (1) to be worn on teeth, in particular designed as a dental splint (2), for intra-oral delivery of one or more agents (7) to a patient,
- the appliance (1) comprising a solid core (4), **characterized in that**
- within a region of interest (13) the core (4) features a multitude of micro-reservoirs (3), which are formed in the core (4).

2. Dental appliance (1) according to claim 1,
- wherein each micro-reservoir (3) is formed by an air pocket (28) of sub-mm size and with a diameter of at least 10 µm, preferably of at least 50 µm, in particular of at least 100 µm,
- in particular wherein each air pocket (28) has been actively formed at a pre-defined location using a separate fabrication step.

3. Dental appliance (1) according to claim 1,
- wherein each micro-reservoir (3) is filled, at least partly, with a filling material (6) that is capable of absorbing and releasing an agent (7), such as a flavor molecule, an antimicrobial or a drug,
- preferably wherein the filling material (6) is loaded with such an agent (7).

4. Dental appliance (1) according to one of the preceding claims,
- wherein the micro-reservoirs (3) are covered by a cap layer (10),
- preferably wherein the cap layer (10) comprises, preferably is made up entirely, of cellulose acetate butyrate (CAB).

5. Dental appliance (1) according to one of the preceding claims,
- wherein the micro-reservoirs (3) each have a diameter of less than 0.5 mm, preferably of less than 0.2 mm,
- most preferably and a depth (5) that is at least 5%, preferably at least 25%, of a thickness of the core (4), and/or
- wherein the depth (5) of each micro-reservoir (3) is larger than 0.05 mm, preferably larger than 0.2 mm or even larger than 0.5 mm,
and/or
- wherein the micro-reservoirs (3) each offer an aspect ratio AR = depth/diameter of AR ≥ 0.2, preferably of AR ≥ 0.5, such that the depth (5) of each micro-reservoir (3) amounts to at least 20%, preferably at least 50%, of the diameter of the micro-reservoir (3).

6. Dental appliance (1) according to one of the preceding claims,
- wherein the micro-reservoirs (3) have been patterned into the core (4) after forming the core (4),
- preferably using an ablative process, most preferably an ablative laser-process, which removes the material of the core (4) at the location of each micro-reservoir (3).

7. Dental appliance (1) according to any of the claims 1 to 5,
- wherein the micro-reservoirs (3) have been formed together with the core (4) in a fabrication step,
- in particular using
- an additive manufacturing technique such as 3D-printing, in particular such that the micro-reservoirs (3) are embedded in the core (4) or
- a forming technique such as hot embossing,
- in particular wherein the core (4) features multiple layers of different materials,
or
- wherein the micro-reservoirs (3) have been formed in a separate outer, in particular 3D-printed, layer (4b) of the core (4) that is formed on a solid inner layer (4a) of the core (4).

8. Dental appliance (1) according to claim 4,
- wherein the cap layer (10) is different from the filling material (6), and
- wherein the cap layer (10) covers and closes each of the micro-reservoirs (3), such that the agent (7) can be loaded and released from the filling material (6) located in the micro-reservoirs (3) only through the cap layer (10),
- preferably wherein the cap layer (10) offers a diffusion rate with respect to an agent (7) that is embedded into the filling material (6) that is at least 50% lower than the corresponding diffusion rate of the filling material (6),
- in particular such that the cap layer (10) forms a diffusion barrier that limits the release rate of each of the micro-reservoirs (3).

9. Dental appliance (1) according to claim 4,
- wherein the cap layer (10) is different from the filling material (6) and covers each of the micro-reservoirs (3), and
- wherein, for each micro-reservoir (3), a corresponding release hole (12), preferably with a diameter of less than 20 µm, is formed in the cap layer (10),
- preferably wherein the cap layer (10) is impermeable to the agent (7) that is embedded into the filling material (6) and/or
- wherein the filling material (6) is a porous material featuring microscopic, in particular sub-micrometer-sized, voids.

10. Dental appliance (1) according to one of the preceding claims,
- wherein a melting temperature of the filling material (6) is
- lower than a glass transition temperature of the material used for the core (4) and/or
- equal or lower than the melting temperature of the cap layer (10),
- in particular such that the core (4) can be thermally fused with the filling material (6) during thermoforming of the dental appliance (1).

11. **A series (30) of dental appliances (1)** for intra-oral delivery of one or more agents (7) to a patient,
- wherein each dental appliance (1) is designed according to claim 1, **characterized in that,**
- the series (30) of dental appliances (1) are all made from common materials and share a common solid core (4),
- the dental appliances (1) employ the same type of micro-reservoirs (3) fabricated in the same way, and
- wherein the dental appliances (1) differ from each other in a geometrical design parameter, in particular
- a spatial density and/or
- an average depth and/or
- a respective volume,
of the micro-reservoirs (3),
- preferably wherein each dental appliance (1) has been fabricated using the method according to the following claim.

12. **A computer-implemented method** for producing a series (30) of different dental appliances (1), in particular a series (30) according to the previous claim, **comprising the following steps:**
- specifying a number of m different numerical values for at least one agent release parameter such as
- a maximum initial release rate in mg/hour and/or
- a total maximum release amount in mg over a time period of 6 hours and/or
- a maximum areal release rate in (mg/cm²)/hour for a particular releasable agent (7) in a digital data set,
- calculating a set of m different design parameters for defining a set of m different micro-reservoir-arrangements (29), in particular using a numerical and empirical model;
- producing said series (30) of m different dental appliances (1), wherein each of the m dental appliances (1) features a micro-reservoir-arrangement (29) according to one of the m different design parameters,
- in particular such that each of the m different dental appliances (1) meets the respective specified release parameter.

13. The computer implemented method according to the previous claim, wherein the digital data set specifying the m different numerical values for the at least one agent release parameter comprises
- a set of patient-specific values, each being based on the specific medical need of a single patient, in particular as specified by a treatment instruction for that patient and/or
- a subset of N treatment-specific-values, each based on a specified dosage of the at least one agent to be delivered to a particular patient within a specified time interval as laid out in a treatment plan for that patient.

14. **Process for fabricating a dental appliance (1),** preferably according to one of the preceding claims, wherein the dental appliance (1) comprises a solid core (4),
**characterized in that**
- a core (4) of the dental appliance is first formed as a core foil (14),
- a multitude of micro-reservoirs (3) are defined in an outer surface (8) of the core (4) using
- an ablative process, preferably an ablative laser-process, which removes the material of the core (4) at the location of each micro-reservoir (3),
or
- a forming process, such as hot embossing,
or
- an additive process, for example molding or 3D-printing of an additional outer layer (4b) of the core (4).

15. **Method for reloading a dental appliance (1),** preferably according to one of the claims 1-10, with a releasable agent (7) such as a flavor, a drug or an antimicrobial agent, **characterized in that**
- the dental appliance (1) is immersed in a reload-liquid (25) containing the agent (7),
- the reload-liquid (25) is heated to an elevated temperature of at least 55°C while the dental appliance (1) is immersed in the reload-liquid (25),
- preferably using an electrically heatable reload-station (26) providing an immersion basin (27) for taking up and heating up the reload-liquid (25).
